# EUROPEAN PATENT APPLICATION

(11) **EP 0 584 714 A1**
(43) Date of publication of application: **02.03.1994**
(21) Application number: 93113225.2
(22) Date of filing: 18.08.1993
(51) Int. Cl.: A61M 5/145

(54) **Medical solution delivery system**

(30) Priority: 21.08.1992 JP 245488/92
(71) Applicant: NISSHO CORPORATION, Osaka-shi, Osaka-fu (JP)
(72) Inventor: Futagawa, Hitoshi, Kusatsu-shi, Shiga-ken (JP); Kikuchi, Toshihiro, Suita-shi, Osaka-fu (JP)
(74) Representative: VOSSIUS & PARTNER

(57) **Abstract**

A medical solution delivery system comprises (a) a cylindrical container (1) having an opening (12) with a flange (14) at one end thereof and a port (11) for a medical solution at the opposite end (13); (b) a plunger (2) having a gasket at one end thereof; (c) a plunger-driving means (3) having an elasticity spring as a source of motive power for forcing the plunger (2) to move toward the port (11) of the container (1); and (d) a flow control means (6) to be connected to the port (11) of the container (1) to deliver the medical solution at a controlled flow rate.

## Description

The present invention relates to a medical solution delivery system and, more particularly, a system suitable for continuously delivering a certain amount of a medical solution to a blood vessel, extraduramater, subcutaneous tissue, or the bladder of a patient by little and little.

In order to administer a very small amount of a medical solution such as a antibiotic agent, or carcinostatic agent, or the like to the blood vessel, bladder, or the like of a patient, there have been proposed various kinds of medical solution delivery systems, as disclosed in JP-A- S50-108790, JP-A- S56-102252, JP-A-H1-135360 and JP-A- H3-170163. Such delivery system comprises an expanded balloon of an elastic material with a medical solution filled therein, a housing for holding the balloon therein, and a flow control means connected to the balloon to control a flow rate of the solution.

In such a system, the medical solution is delivered from the balloon little by little by means of shrinkage thereof. In other wards, the balloon is used as a container for storing a medical solution and as a motive power source for delivering the solution therefrom.

Accordingly, it is inevitable with such a medical solution delivery system to avoid influences of a material used for production of the balloon. The balloon is made of an elastomeric gum rubber, the force applied to the medical solution varies with time during injection, thus making it impossible to delivery a fixed amount of the medical solution at a uniform flow rate. In addition, it is required to use an elastomeric gum rubber having no problem caused by elution of chemical substances.

It is therefore an object of the present invention to provide a medical solution delivery system which enables to deliver a fixed amount of a medical solution at a uniform flow rate.

Another object of the present invention is to provide an inexpensive medical solution delivery system which is simple in structure, easy to operate, and small in the number of components, and which is free from elution of chemical substances into a medical solution.

According to the present invention, these and other objects are solved by providing a medical solution delivery system comprising:
(a) a cylindrical container having a port for a medical solution at one end thereof and a flanged opening at the opposite end;
(b) a plunger having a gasket at one end thereof, said plunger being movably arranged in said container so that the gasket is in sliding contact with an inside wall of said container;
(c) a plunger-driving means having an elasticity as a source of motive power for forcing the plunger to move toward the port of the container, said plunger-driving means being connected at one end thereof to said plunger and at the opposite end to said container; and
(d) a flow control means to be connected to the port of said container to deliver the medical solution at a controlled flow rate.

As the plunger-driving means used as a motive power source for the delivery system, there may be used those such as constant force springs, rubber strings, coil springs and the like.

In use, a medical solution is firstly drawn into the container in the same manner as a well-known syringe and then the flow control means is connected to the port of the container at one end thereof and at the opposite end to a catheter. After removing the air from the container by applying a constant force with the plunger-driving means, the catheter is inserted into the blood vessel of a patient and the plunger is forced by the plunger driving means to move toward the front end of the container 1 so that the medical solution in the container is delivered therefrom through the port and then injected into the blood vessel at a certain flow rate determined by the flow control means.

The above and other objects, features and advantages of the present invention will become apparent from the following description taken in conjunction with the preferred embodiments thereof with reference to the accompanying drawings.
Fig. 1 is a plan view of a medical solution delivery system embodying the present invention;
Fig. 2 is a cross sectional view of the medical solution delivery system, taken along a line X-X in Fig. 1;
Fig. 3 is a plan view illustrating the essential part of a medical solution delivery system of another embodiment of the present invention;
Fig. 4 is a cross sectional view of the medical solution delivery system, taken along a line Y-Y in Fig. 3; and
Fig. 5 is a sectional view of a medical solution delivery system illustrating still another embodiment of the present invention.

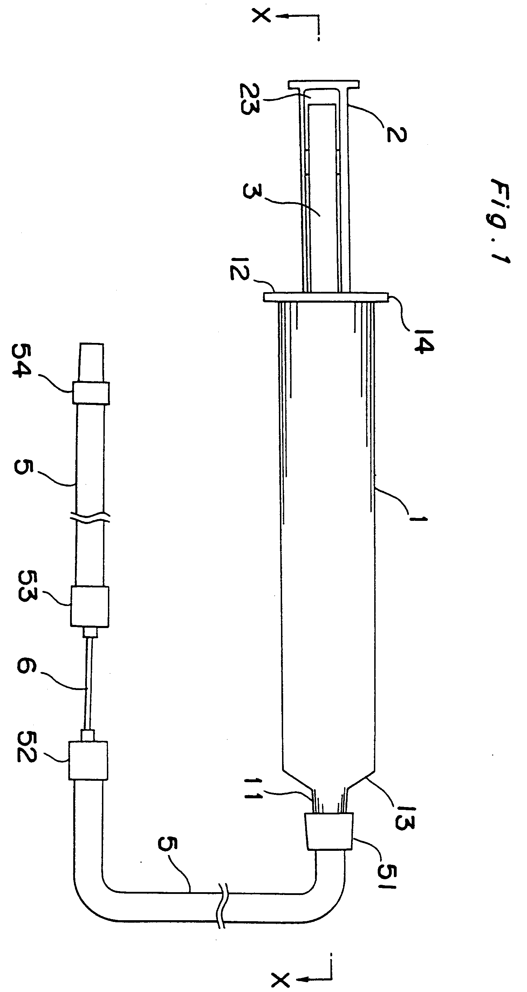

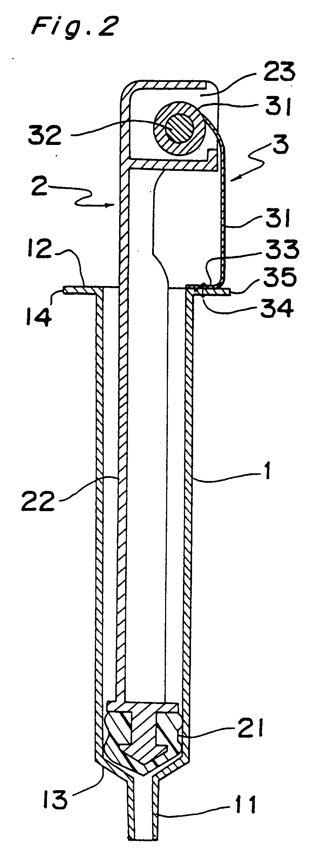

Referring now to Figs. 1 and 2, there is shown a medical solution delivery system of the present invention, which comprises a cylindrical container 1 having a port 11 at one end 13 thereof and a flanged opening 12 at the opposite end, a plunger 2 slidably arranged therein, a plunger-driving means 3 having an elasticity as a source of motive power for forcing the plunger 2 to move smoothly within the container 1 toward the port 11 thereof, and a flow control means 6 used for controlling a flow rate of a medical solution and provided with a connecting means such as a flexible connecting tube 5.

The container 1 is a cylindrical receptacle, usually made of glass or a transparent synthetic resin such as polypropylene, polyester, poly(4-methylpentene-1), polycarbonate, or the like, with a narrow mouth serving as the port 11, the part below swelling out and then continuing straight down to the lower end having a flange 14. Fixed to the flange 14 is a plunger-driving means mentioned below.

The plunger 2 used in combination with the container 1 comprises a plunger rod 22 with a cross section of a circular arc and a gasket 21 attached to a front end thereof. The plunger rod 22 is usually made of glass or a synthetic resin such as polypropylene, polyethylene, polyester, polycarbonate, or the like, and provided at the rear end thereof with a spring chamber 23 for holding a part of the plunger-driving means therein. The gasket 21 is usually made of butyl rubber or an olefin elastomer to seal an contacting area between the plunger 2 and container 1 against leakage. Thus, the container 1 and the plunger 2 combined therewith constitute a syringe or a plunger device for injecting or withdrawing medical solutions, like as a well-known syringe.

The plunger-driving means 3 comprises a constant force spring 31 such as CONSTON (Trademark, Sanko Hatsujo Corporation). The spring 31 is fixed at one end thereof to a drum 32 and wound around the same. A part of the spring 31, wound on the drum 32, is arranged in the spring chamber 23 on the rear side of the plunger 2 and brought into contact with the rear end of the plunger 2. The drum 32 may be rotatably arranged in the spring chamber 23 of the plunger 2 by means of a shaft (not illustrated). The opposite end or free end of the spring 31 is fixed to the flange 14 of the container 1 by a fixing member such as a bolt or a pin 34. To that end, the spring 31 is bent at the opposite end thereof to form a fixing portion 33 having a hole 35 through which the pin 34 is fixed to the flange 14 of the container 1.

The flow control means 6 is connected to the port of the container 1 by means of a connecting tube 5 to control a flow rate of the medical solution to be delivered from the container 1. The connecting tube 5 is separated into two parts, i.e., an upstream part and a downstream part by means of the flow control means 6. The upstream part of the connecting tube 5 is connected at one end thereof to the container 1 by a connector 51 and at the opposite end to one end of the flow control means 6 by a connector 52, whereas the downstream part of the connecting tube 5 is connected at one end thereof to the opposite end of the flow control means 6 by a connector 53 and at the other end to a connector 54 used for attachment of a catheter (not illustrated). However, the connecting tube 5 is unnecessarily required for the delivery system of the present invention. The flow control means 6 may be connected to the port 11 of the container 1.

The flow control means 6 may have any configuration depending on the time required for administration of the medical solution or on an amount of the medical solution per unit time. Examples of preferred flow control means are those comprising a narrow tube provided with a very small bore as disclosed in the Japanese Patent Gazettes JP-A- S64-70069 or JP-A-H1-135359, or those comprising a pipe with a very small diameter as disclosed in JP-A- H2-11159 or JP-A- H3-140163.

To make the above medical solution delivery system ready for use, the flow control means 6 is removed first from the port 11 of the container 1 and then a medical solution to be transfused into a vein of a patient is drawn into the container 1 by pulling the plunger 2 toward the flanged opening 12 of the container 1. This may be done with ease after the wound part of the spring 31 has been taken off from the spring chamber 23 of the plunger 2. Of course, the wound part of the spring 31 is put in the spring chamber 23 of the plunger 2 after the medical solution has been drawn into the container 1. In that case, the spring 31 is deflected through a certain displacement, thereby storing energy.

The flow control means 6 is then connected to the port 11 of the container 1 by the tube 5 at one end thereof and at the opposite end to a catheter by the connector 54. In that case, the connecting tube 5 is closed by a suitable closing means such as a clamp (not illustrated in the drawings).

Then, the closing means is removed from the tube 5 so that air in the container 1 and the tube 5 is expelled therefrom as the force is applied to the plunger 2 by the plunger-driving means 3. After the air has been removed from the deliver system, the tube 5 or the catheter is clamped again by the clamping means and then the catheter is inserted into the vein of the patient.

Next, the clamping means is removed form the tube 5 or the catheter so that the plunger 2 is forced again by the constant force spring 31 to move towards the front side of the container 1. Thus, the medical solution in the container 1 is delivered therefrom through the port 11 and injected into the vein of the patient at a fixed flow rate determined by the flow control means 6.

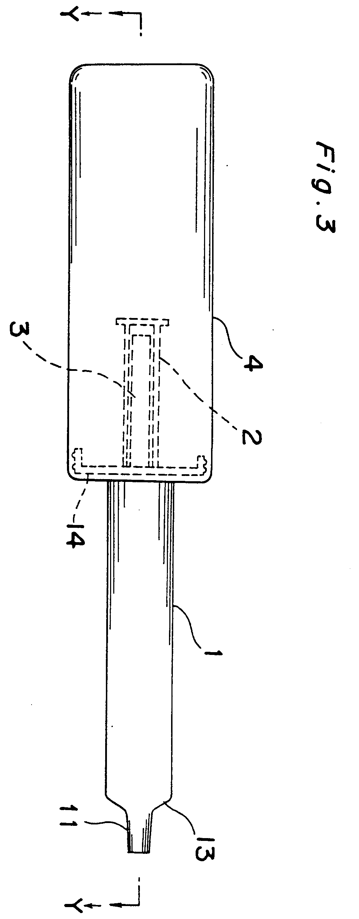

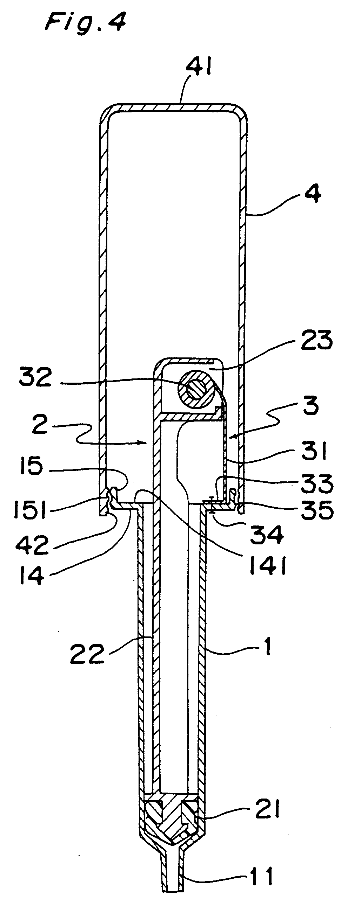

If the medical solution is to be administered to a patient over a long time of period, the sliding movement of the plunger may be disturbed by motion of a patient. For example, if the medical solution delivery system is set under clothes of the patient, the plunger 2 may be disturbed its movement by the clothes. Also, the medical solution delivery system may be buried beneath the body of the patient then turning in bed. In order to avoid such obstructions due to the clothes or motion of the patient, it is preferred to protect the plunger and the plunger-driving means from the influence of motion of the patient with a cap 4 as shown in Fig. 3 and 4.

Referring now to Figs. 3 and 4, there is shown another embodiment of the medical solution delivery system of the present invention. This system further comprises a cap 4 in addition to a cylindrical container 1, a plunger 2, a plunger-driving means 3, and a flow control means 6. The plunger 2 is provided at the flange 14 thereof with a cylindrical portion 15 having a male screw 151 so that a cap 4 may be screwed thereon. The fixing portion 33 of the spring 31 is fixed to an inside wall 141 of the flange 14 surrounded by the cylindrical portion 15.

The cap 4 may be attached to the cylindrical container 1 in various manners such as screw-mounting, or press-fitting, fusion-mounting, or bolts.

For example, the cap may be fixed to the flange 14 of the container 1 by a bolt (not shown). In that case, it is required to provide a hole in the closed end 41 of the cap 4 to allow the plunger 2 to be moved backward by an actuating rod which may be inserted into the cap through the hole when drawing a medical solution in the container.

If the cap 4 is to be fitted on the cylindrical portion 15 of the flange 14, the cylindrical portion 15 is provided with an annular rib and the cap 4 is provided with a corresponding annular groove or a rib.

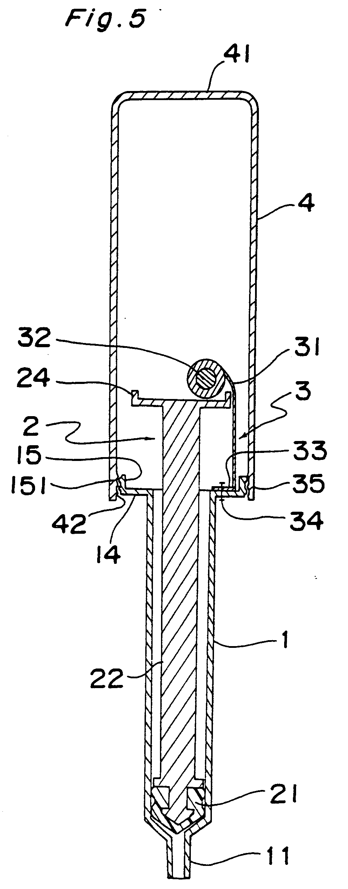

Fig. 5 shows a modified form of the medical solution delivery system of Figs. 3 and 4, which comprises a solid plunger having a structure similar to that of a commercially available syringe, and a cylindrical container shown in Figs. 3 and 4. In this embodiment, the wound part of the constant force spring 3 is brought into contact with the flange 24 of the plunger 2, while the free end of the constant force spring 3 is fixed to the inside wall 141 of the flange 14 surrounded by the cylindrical portion 15 of the cylinder 1. The cap 4 is screwed on the cylindrical portion 15 of the cylinder 1.

As will be understood from the above, the medical solution delivery system of the present invention is simple in structure and small in the number of components, thus making it possible to provide inexpensive medical solution delivery systems. Also, the system is free from elution of chemical substances as the container is of glass or a polyolefine resin. By employing the constant force spring, it is possible to provide a medical solution delivery system capable of injecting a medical solution accurately at a well-controlled flow rate. Further, it is possible to inject the medical solution into the patient safely and certainly since the plunger and plunger-driving means can be protected from external influences such as, for example, pressures caused by movement of the body of the patient.

Although the present invention has been fully described in connection with the preferred embodiments thereof with reference to the accompanying drawings, it is to be noted that various changes and modifications are apparent to those skilled in the art. Such changes and modifications are to be understood as included within the scope of the present invention as defined by the appended claims unless they depart therefrom.

## Claims

1. A medical solution delivery system comprising:
(a) a cylindrical container having an opening with a flange at one end thereof and a port for a medical solution at the opposite end;
(b) a plunger having a gasket at one end thereof, said plunger being movably arranged in said container so that the gasket is in sliding contact with an inside wall of said container;
(c) a plunger-driving means having an elasticity as a source of motive power for forcing the plunger to move toward the port of the container, said plunger-driving means being connected at one end thereof to said plunger and at the opposite end to said container; and
(d) a flow control means to be connected to the port of said container to deliver the medical solution at a controlled flow rate.

2. The medical solution delivery system claimed in claim 1 wherein said plunger-driving means comprises a constant force spring wound around a drum, said constant force spring being fixed at a free end thereof to the flange of said container, while the wound part of said constant force spring being held in a spring chamber provided at the rear end of the plunger.

3. The medical solution delivery system claimed in claim 2 wherein said constant force spring is wound around a drum having a shaft, the shaft being rotatably arranged in the spring chamber.

4. The medical solution delivery system as claimed in any of claims 1 to 3
wherein said flange of the container is provided with a cap for protecting the plunger and the plunger-driving means from external forces.

5. The medical solution delivery system claimed in claim 4 wherein said cap is screwed on the flange of the container.

6. The medical solution delivery system claimed in claim 1 wherein said plunger-driving means comprises a constant force spring wound around a drum, the free end of said constant force spring being fixed to the flange of said container, the wound part of said constant force spring being brought into contact with the rear end of the plunger, the opening of the container is provided with a cap for protecting the plunger and the plunger-driving means from external forces.
